# EUROPÄISCHE PATENTSCHRIFT

(11) **EP 4 373 382 B1**
(45) Veröffentlichungstag und Bekanntmachung des Hinweises auf die Patenterteilung: **09.10.2024**
(21) Anmeldenummer: 23789571.9
(22) Anmeldetag: 10.10.2023
(51) Int. Cl.: A61B 1/005, A61B 1/31, A61B 1/018, A61B 1/05

(54) **ENDOSKOP MIT DREHANTRIEB**
ENDOSCOPE WITH ROTARY DRIVE
ENDOSCOPE A ENTRAÎNEMENT ROTATIF

(30) Priorität: 11.10.2022 DE 102022126400
(43) Veröffentlichungstag der Anmeldung: 29.05.2024
(73) Patentinhaber: Bob, Konstantin, 69469 Weinheim (DE)
(72) Erfinder: GRÜNDL, Andreas, 82319 Starnberg (DE); BOB, Konstantin, 69469 Weinheim (DE)
(74) Vertreter: Winter, Brandl - Partnerschaft mbB
(86) Internationale Anmeldenummer: PCT/EP2023/078082
(87) Internationale Veröffentlichungsnummer: WO 2024/079135

(56) Entgegenhaltungen:
- WO-A1-2021/079221
- JP-A- 2009 112 536
- US-A1- 2006 167 342
- US-A1- 2013 253 271

## Beschreibung

Die vorliegende Offenbarung betrifft ein Endoskop mit einem im Wesentlichen schlauchförmigen proximalen Schaft, welcher zum Einführen in einen Patientenkörper ausgebildet ist und sich entlang einer Schaftachse erstreckt, einem im Wesentlichen schlauchförmigen Lenkabschnitt, welcher distal vom Schaft angeordnet ist und bezüglich der Schaftachse abkrümmbar ist, einem Endoskopkopf, welcher distal vom Lenkabschnitt angeordnet ist und eine Optik und einen Arbeitskanalausgang aufweist, und einem Drehmechanismus, welcher dazu konfiguriert ist, den Lenkabschnitt bezüglich des Schafts um die Schaftachse zu drehen.

### Hintergrund der Offenbarung

Endoskope sind medizinische Arbeitsgeräte zur visuellen Exploration von Hohlräumen in einem Patientenkörper. Sie weisen grundsätzlich optische Einrichtungen am distalen, d.h. Anwender-abgewandten bzw. Patientenkörper-zugewandten, Endoskopkopf, einen proximalen, d.h. Anwender-zugewandten, Endoskopbedienabschnitt oder Endoskopgriff und einen flexiblen oder biegesteifen (starren) Schaft auf, welcher den Endoskopkopf und den proximalen Endoskopbedienabschnitt oder Endoskopgriff verbindet. In der Regel hat das Endoskop ferner einen Arbeitskanal, welcher sich vom proximalen Endoskopbedienabschnitt durch den Schaft zu einem am Endoskopkopf gebildeten Arbeitskanalausgang erstreckt und das extrakorporale Einführen und Verwenden eines medizinischen Instruments wie z.B. einer Zange, Schere, Nadel, Schlinge, Messer und dergleichen ermöglicht. Viele Endoskope haben ferner Deflectings, d.h. einen steuerbaren Schaftabschnitt, welcher zwischen dem Schaft und dem Endoskopkopf angeordnet ist, um ein Steuern des Endoskops in einem Patientenhohlraum zu ermöglichen.

Derartige Endoskope können wahlweise mit zusätzlichen Fähigkeiten versehen werden, etwa indem am distalen Endoskopende/Endoskopkopf eine Kappe oder Hülse auf den Endoskopkopf radial außenseitig aufgesetzt wird, die mit bestimmten Funktionen/Funktionselementen versehen oder ausgerüstet ist, wodurch das Endoskop nicht nur zur Exploration und/oder als Zugang für therapeutische Anwendungen sondern auch selbst als ein minimalinvasives Instrument zur Ausführung eines chirurgischen Vorgangs genutzt werden kann. Alternativ hierzu ist es aber auch vorgesehen, spezielle Endoskope für ganz bestimmte medizinische Anwendungen mit derartigen Fähigkeiten integral auszustatten, wobei derartige Spezialanfertigungen jedoch nur für diese besondere Anwendung geeignet sind.

Verschiedene diagnostische und/oder therapeutische Verfahren erfordern beispielsweise eine Bildgebung und/oder bei Bedarf therapeutische Techniken am Gallen- und/oder Bauchspeicheldrüsengang sowie den Lebergängen des Patienten. Da die Vatersche Papille, welche den gemeinsamen Austritt von Gallen- und Bauchspeicheldrüsengang in das Duodenum bildet, seitlich in das Duodenum hineinragt, sind herkömmliche prograde (in Endoskop-Längsrichtung blickende) Endoskope für das Heran- und Einführen von chirurgischen Instrumenten in den Gallenkanal ungeeignet, da nicht genügend Schwenkraum im engen Duodenum (Durchmesser 3 bis 4 cm) vorhanden ist, um deren prograde Optik sowie den Arbeitskanal in eine seitwärts blickende Position auszurichten, da ein typischer Biegeradius solcher Geräte bei etwa 6 cm liegt. Dieser Biegeradius bzw. diese Verschwenkung wird bei herkömmlichen prograden Endoskopen häufig durch sogenannte "Deflectings", d.h. zwischen dem Schaft und dem Endoskopkopf angeordnete, insbesondere dem Endoskopkopf unmittelbar in Richtung proximal vorgeordnete, aktiv abkrümmbare Schaftabschnitte erzielt.

### Stand der Technik

Aus dem Stand der Technik (bspw. der US 2010/228086 A) sind zu diesem Zweck speziell angefertigte Duodenoskope bekannt, welche eine laterale (seitwärts blickende) oder retrograde (rückblickende) Optik (auch "Seitoptik" genannt) sowie einen seitwärts gerichteten bzw. sich öffnenden Arbeitskanal aufweisen. Alternativ hier kann an einem prograden (in Axialrichtung sich öffnenden) Ausgang des Arbeitskanals solcher Duodenoskope ein sogenannter Albarranhebel vorgesehen sein, der durch manuell bewirktes Verschwenken ein gezieltes Führen/Umlenken eines im Arbeitskanal geführten Instruments ermöglicht. Durch die seitwärts gerichtete Anordnung der Funktionseinheiten insbesondere Optik und Beleuchtung am Endoskopkopf wird eine Bildgebung und Behandlung im Bereich des Duodenums unter optimaler Nutzung des zur Verfügung stehenden Raums ermöglicht.

Derartige Endoskope mit Seitoptik sind jedoch sehr aufwendig und teuer in ihrer Fertigung und werden deshalb bislang als wiederverwendbare Geräte entwickelt und hergestellt. Der gekrümmte Arbeitskanal solcher Endoskope sowie die komplexe und hinterschnittreiche Konstruktion des Albarranhebels haben sich in der Praxis zudem als schwer sterilisierbar erwiesen. Dadurch ist ein Risiko für eine Kontamination des Endoskops und dadurch bedingt die Kontamination einer Operationsstelle oder eines Hohlraums eines Patienten erhöht, bei welchem das Endoskop eingesetzt wird. Dadurch kann es an der Operationsstelle oder in dem Hohlraum zu schwerwiegenden Entzündungen bis hin zu einer Sepsis kommen. Weiter haben solche Geräte den Nachteil, dass sie nur für wenige, sehr spezifische Eingriffe im Bereich des Duodenums eingesetzt werden können, da weder die Optik noch der Arbeitskanal in prograde Richtung gerichtet werden können. Die Navigation im Patientenkörper insbesondere in engen Patientenhohlräumen ist somit mit seitwärts blickenden Endoskopen im Allgemeinen eher schwierig.

Des Weiteren ist aus DE 10 2018 110 620 A1 ein Abklappmechanismus zur abklapp- oder abwinkelbaren Halterung eines Endoskopkopfes bekannt, mit einer Anzahl axial aufeinanderfolgender und mittels eines Betätigungselements aktiv d.h. manuell kontrolliert gegeneinander winkelverstellbarer Segmente. Durch diese Konstruktion ist es möglich, den distalen Endoskopkopf in einem sehr engen Radius in eine Radialausrichtung zu verschwenken, derart, dass die distale Stirnseite des Endoskopkopfs nicht oder nur geringfügig über den Außenumfang des Endoskopschafts radial vorragt. Nachteilig an diesem Mechanismus ist jedoch, dass ein solcher Mechanismus nur dazu geeignet ist, in einer einzigen bestimmten Richtung abzuklappen und somit die Anwendungsbreite des zugehörigen Endoskops beschränkt ist. Zudem kann es problematisch sein, wenn der Abklappmechanismus an der zu begutachtenden Stelle im Patientenhohlraum falsch ausgerichtet ist.

WO 2012/070781 A2 offenbart einen Drehmechanismus, der einen flexiblen Endoskopschaft mit einem distal dazu angeordneten Biegemechanismus um die Schaftachse zueinander verdrehbar verbindet, wobei verschiedene Antriebe bereitgestellt sind. Diese Antriebe sind jedoch sehr aufwändig und benötigen viel Platz und/ oder sind, wie zum Beispiel im Fall eines Antriebs über ein Seil, fehleranfällig und fragil.

Ein weiterer Drehmechanismus, der einen Seilzug verwendet, ist aus der WO 2021/079 221 A1 bekannt. Bei diesem Drehmechanismus ist das Seil an einer Drehhülse über Ösen umgelenkt.

Weiterer Stand der Technik ist aus der DE 10 2004 058 929 A1 oder der JP 2009 112 536 A bekannt.

### Zusammenfassung der Offenbarung

Die der Offenbarung zugrundeliegende Aufgabe ist es, Nachteile des Stands der Technik zu vermeiden oder zu reduzieren. Insbesondere soll ein Endoskop mit einem steuerbaren Endoskopkopf und einem einfachen, robusten Drehantrieb bereitgestellt werden.

Die Aufgabe wird gelöst durch ein Endoskop nach Anspruch 1. Vorteilhafte Ausgestaltungen sind Gegenstand der Unteransprüche.

Genauer wird die Aufgabe gelöst durch ein Endoskop mit einem im Wesentlichen schlauchförmigen Schaft, welcher zum Einführen in einen Patientenkörper ausgebildet ist und sich entlang einer Schaftachse erstreckt, einem Endoskopkopf, welcher distal vom Schaft angeordnet ist, bezüglich der Schaftachse abkrümmbar oder abklappbar ist und eine Kamera sowie einen Ausgang eines Arbeitskanals aufweist, einem Drehmechanismus, welcher zwischen dem Endoskopkopf und dem Schaft angeordnet und dazu konfiguriert ist, den Endoskopkopf bezüglich des Schafts um die Schaftachse zu drehen. Der Drehmechanismus hat eine Basishülse, welche bezüglich der Schaftachse drehfest mit dem Schaft verbunden ist, und eine Drehhülse, welche bezüglich der Schaftachse drehfest mit dem Endoskopkopf oder einem zwischenliegenden Abschnitt verbunden ist, wobei die Drehhülse und die Basishülse ineinander geschachtelt sind. Der Drehmechanismus hat ferner ein erstes Seil mit einem distalen Seilabschnitt, welcher an der Drehhülse derart befestigt ist, dass das erste Seil um die Drehhülse aufwickelbar oder abwickelbar ist, wobei die Basishülse in ihrer Seitenwand oder an/ in einer der Drehhülse radial gegenüberliegenden (d.h. entgegengesetzten) Fläche der Seitenwand einen ersten Führungsabschnitt in Form einer rillen- oder schlitzförmigen Aufnahme bildet, in welchem das erste Seil geführt ist. Insbesondere ist die rillen- oder schlitzförmige Aufnahme zumindest an einer der Drehhülse zugewandten Seite geschlossen, z.B. durch einen integralen Wandabschnitt der Basishülse und/ oder durch einen zur Basishülse separat ausgebildetes, aber fest damit verbundenen Wandabschnitt. Ein solcher separater, aber fest mit der Basishülse verbundener Wandabschnitt kann z.B. ein in die rillen- oder schlitzförmige Aufnahme der Basishülse (z.B. durch Löten, Kleben oder dergleichen) fest eingesetzter Wandabschnitt, wie z.B. ein Röhrchen, in dem das erste Seil geführt ist, sein. An einem distalen Ende des ersten Führungsabschnitts bildet die Basishülse einen ersten distalen Durchgang, durch welches das erste Seil aus dem ersten Führungsabschnitt zur Drehhülse geführt ist. D.h. der Durchgang verbindet die rillen- oder schlitzförmige Aufnahme bzw. ggf. ein darin eingesetztes Element, wie das Röhrchen, mit einer der Drehhülse zugewandten Seite des Basiselements, sodass das Seil aus der rillen- oder schlitzförmige Aufnahme durch den Durchgang zur Drehhülse geführt wird. Insbesondere kann der Durchgang ein Loch in dem integralen oder dem separaten, aber fest mit der Basishülse verbundenen Wandabschnitt sein. Alternativ kann der Durchgang durch eine Lücke zwischen einem distalen Ende der rillen- oder schlitzförmige Aufnahme und einem distalen Ende des darin eingesetzten, separaten, Wandabschnitts (z.B. einem distalen Ende des Röhrchens) sein. Dort kann das erste Seil um die Drehhülse gewickelt sein.

In anderen Worten ausgedrückt ist ein Endoskop bereitgestellt, welches einen Schaft und einen distalen Endoskopabschnitt mit einem abkrümmbaren oder abklappbaren Endoskopkopf und einem Drehantrieb zwischen dem Schaft und dem distalen Endoskopabschnitt aufweist. Der Drehantrieb ist ein Seilantrieb, welcher darauf basiert, dass ein erstes Seil bereitgestellt ist, welches in Umfangsrichtung auf eine Drehhülse (auch als Spule bezeichenbar) aufgewickelt ist, und durch einen Zug am ersten Seil ein Abwickeln des ersten Seils und dadurch eine Drehung der Drehhülse erreicht wird. Die Drehhülse ist hierzu drehbar und bevorzugt axialfest an einer Basishülse gelagert, welche den Drehmechanismus mit dem Schaft verbindet.

Angaben wie "axial", "Umfangsrichtung" und "radial" beziehen sich stets auf die Schaftachse. Der Schaft kann starr oder flexibel sein. Der Endoskopkopf kann ferner eine Beleuchtungseinrichtung und/oder eine Spüleinrichtung und/oder weitere Elemente aufweisen, welche mit zugehörigen Leitungen verbunden sind. Ineinander geschachtelt heißt, dass die beiden Hülsen (Drehhülse und Basishülse) sich axial zumindest teilweise überlappen bzw. dass eine der beiden Hülsen in die andere der beiden Hülsen eingesteckt ist.

Der Drehmechanismus wird betätigt, indem am ersten Seil gezogen wird, welches um die Drehhülse gewickelt ist. Dadurch wickelt sich das Seil von der Drehhülse ab und treibt diese in Umfangsrichtung, d.h. drehend, an. Der Endoskopkopf ist insbesondere bezüglich der Schaftachse abklappbar oder krümmbar, insbesondere zu genau einer Seite hin (d.h. in einer einzelnen Radialrichtung).

Der Drehmechanismus des vorstehenden Endoskops hat eine besonders einfache Konfiguration und ist somit kostengünstig herzustellen. Ferner ist der Drehmechanismus reibungsarm und somit einfach zu bedienen. Zudem ist das erste Seil besonders sicher geführt und umgelenkt, sodass es sicher geführt wird. Ein Risiko, dass das Seil reißen könnte, ist somit minimiert.

Bevorzugt ist ein zweites Seil bereitgestellt, welches durch einen zweiten Führungsabschnitt in Form einer rillen- oder schlitzförmigen Aufnahme in der Basishülse in Richtung distal verlaufend geführt ist und in einer zum ersten Seil entgegengesetzten Umfangsrichtung um die Drehhülse gewickelt oder wickelbar ist. Bevorzugt bildet die Basishülse am distalen Ende des zweiten Führungsabschnitts einen zweiten distalen Durchgang, welcher in Umfangsrichtung zum ersten Durchgang beabstandet ist und durch welchen das zweite Seil hindurchgeführt ist.

In anderen Worten ausgedrückt verlaufen der zweite Führungsabschnitt und das zweite Seil spiegelbildlich zum ersten Führungsabschnitt und dem ersten Seil. Dadurch kann besonders einfach und zuverlässig ein Antrieb der Drehhülse in beide Drehrichtungen bereitgestellt werden. D.h., je nachdem, an welchem der beiden Seile (dem ersten oder zweiten Seil) gezogen wird, wird die Drehhülse in unterschiedliche Richtungen gedreht. Zumindest eines der beiden Seile ist bei dieser Ausgestaltung stets um die Drehhülse gewickelt. In einer neutralen Position sind beide Seile zu zumindest 180° um die Drehhülse gewickelt.

Bevorzugt ist das erste und/oder das zweite Seil dazu ausgebildet und dimensioniert, dass es mindestens um 360° um die Drehhülse gewickelt oder wickelbar ist. Somit kann eine vollständige Drehung des Endoskopkopfs erreicht werden. Die Drehhülse kann ein oder mehrere Befestigungslöcher, insbesondere Radialbohrungen, aufweisen, in welchen das erste und/oder das zweite Seil befestigt ist. Dies ermöglicht eine einfache und sichere Befestigung der Seile an der Drehhülse.

Bevorzugt ist das erste und/oder das zweite Seil jeweils in einem Kapillarrohr geführt, welches im oder am ersten bzw. zweiten Führungsabschnitt der Basishülse im Wesentlichen lagefest angebracht ist. Dadurch kann das erste und/oder zweite Seil besonders sicher im jeweiligen Führungsabschnitt gehalten werden. Ein Herausspringen aus dem jeweiligen Führungsabschnitt unter Zug und ein dadurch bedingtes Verklemmen zwischen der Basishülse und einem anderen Bauteil kann somit vermieden werden. Das Kapillarrohr kann z.B. eine Wandstärke von 0,4 mm haben und im ersten bzw. zweiten Führungsabschnitt befestigt, z.B. eingeklebt oder eingelötet sein.

Bevorzugt ist der erste und/oder zweite Führungsabschnitt an einer radialen Außenseite der Basishülse offen, insbesondere entlang seiner gesamten Läge. Dadurch ist eine Montage des ersten bzw. zweiten Seils und ggf. der Kapillarrohre im jeweiligen Führungsabschnitt besonders einfach.

Bevorzugt verläuft zumindest ein Abschnitt des ersten und/ oder zweiten Führungsabschnitts unmittelbar proximal zum ersten bzw. zweiten Durchgang schräg oder quer zur Schaftachse. Dies ermöglicht eine besonders schonende Umlenkung des jeweiligen Seils, sodass Kräfte, welche beim Zug am Seil aufgrund einer Umlenkung der Kraft von distaler in Umfangsrichtung durch das Seil besser abgestützt werden. Ein Risiko, dass das jeweilige Seil reißen könnte, wird somit minimiert. Bevorzugt verläuft ein proximaler Abschnitt des ersten und/oder zweiten Führungsabschnitts parallel zueinander und zur Schaftachse. Die proximalen Abschnitte können auch miteinander verbunden sein und durch eine gemeinsame nut-, rillen- oder schlitzförmige Aufnahme gebildet sein. Bevorzugt entfernen sich in einem distalen Bereich der Führungsabschnitte (d.h. jeweils der Abschnitt des ersten und/ oder zweiten Führungsabschnitts unmittelbar proximal zum ersten bzw. zweiten Durchgang) die Führungsabschnitte in Umfangsrichtung voneinander. In andere Worten verläuft bevorzugt der zweite Führungsabschnitt zumindest im Abschnitt unmittelbar proximal zur zweiten Durchgangsöffnung schräg oder quer zur Schaftachse mit einer zum ersten Führungsabschnitt entgegengesetzten Neigung bzw. Umfangsrichtung. Ein Übergang zwischen dem parallel zur Schaftachse verlaufenden Bereich und dem schräg oder quer zur Schaftachse verlaufenden Bereich des ersten und/ oder zweiten Führungsabschnitts kann kurven- oder knickförmig ausgestaltet sein.

Bevorzugt bildet der erste und/ oder zweite Führungsabschnitt jeweils an seinem proximalen Ende einen proximalen Durchgang, in welchem das erste bzw. zweite Seil aus dem ersten bzw. zweiten Führungsabschnitt nach radial innen geführt ist.

Bevorzugt bildet die Drehhülse oder ggf. auch die Basishülse an einer bestimmten Axialposition, welche einer Axialposition der ersten und zweiten Durchgänge entspricht, eine Antriebsrille oder Ringnut, welche zur Aufnahme des ersten und zweiten Seils ausgebildet ist. Somit können die Seile an einer bestimmten Position aufgewickelt werden und laufen nicht Gefahr, sich zwischen den beiden Hülsen zu verklemmen. Zudem kann somit besonders einfach Platz für eine Befestigung des ersten und/oder zweiten Seils an der Drehhülse bereitgestellt werden. D.h., das erste und/ oder zweite Seil ist bevorzugt innerhalb der Antriebsrille oder Ringnut an der Drehhülse befestigt.

Bevorzugt ist die Basishülse eine radial äußere Hülse ist und die Drehhülse eine radial innere Hülse. Dies ermöglicht eine besonders einfache Montage. Zudem ist ein Abschnitt des Endoskops, dessen radial äußere Elemente sich drehen und ggf. Gewebe des Patienten schädigen könnten, minimiert. Der Drehmechanismus kann ferner eine Schutz- oder Deckhülse oder Schutz- oder Deckfolie als eine radial äußerste Schicht haben. Dadurch kann der erste und/ oder zweite Führungsabschnitt radial außenseitig abgedeckt oder verschlossen werden, um eine Kontamination oder ein Einbringen von Störkörpern in den Drehmechanismus zu unterbinden sowie eine Gefahr, das Gewebe zu verletzen, zu minimieren.

Bevorzugt hat das Endoskop elektrische, hydraulische und/ oder leere Leitungen sowie einen Arbeitskanal (d.h., Schläuche und Kabel), welche sich durch den Schaft und den Drehmechanismus zum oder durch den Endoskopkopf erstrecken. Insbesondere stellt der Drehmechanismus an seinem proximalen Bereich, insbesondere angrenzend an den Schaft, einen Halte- oder Positionierungsring bereit, welcher eine Lage der Leitungen bezüglich der Drehhülse bestimmt. Auf diese Wiese kann ein Überkreuzen der Schläuche und Kabel beim Drehen des Drehmechanismus verhindert werden. In anderen Worten wird eine geordnete Verlegung der Schläuche und Kabel erreicht.

Bevorzugt bildet der Schaft an seinem distalen Ende einen Hohlraum, welcher sich in Richtung distal öffnet und derart dimensioniert ist, dass Platz für eine Drehung und/ oder Umfangsbewegung der Leitungen und des Arbeitskanals ist, welche durch den Schaft zum Endoskopkopf verlaufen und sich in einem distalen Bereich zusammen mit der Drehhülse drehen bzw. dass die Leitungen zwischen verschiedenen, von einer Drehstellung der Drehhülse abhängigen Positionen beweglich sind. Somit kann eine besonders reibungsarme, wenig störungsanfällige Bedienung gewährleistet werden.

Bevorzugt hat der Schaft zumindest an seinem distalen Ende eine nut-, rillen- oder schlitzförmige, sich in Richtung distal öffnende Seilaufnahme, in welcher das erste und/ oder das zweite Seil geführt ist. D.h. das erste und/ oder zweite Seil wird in einem (radialen) Mantelbereich des Schafts in den Schaft eingeführt. Auf diese Wiese kommen das erste und/ oder zweite Seil, welche sich nicht zusammen mit der Drehhülse drehen, nicht den sich drehenden/ bewegenden Leitungen in die Quere und eine besonders reibungsarme, wenig störungsanfällige Bedienung kann gewährleistet werden. Das erste und/ oder zweite Seil kann innerhalb des Schafts in einer Hülle geführt sein. Dadurch können Differenzlängen der Seile vermieden werden, welche sich beim Krümmen des Schafts ergeben könnten. Bevorzugt sind ferner die Leitungen in einem distalen Schaftabschnitt in Umfangsrichtung beweglich sind und mit einer Gleitschicht oder einer Gleithülle ummantelt. Dies ermöglicht es weiter, Reibung zu minimieren, welche durch das Drehen und Bewegen der Leitungen und des Arbeitskanals relativ zum distalen Schaftbereich entstehen. Es kann also eine besonders reibungsarme, wenig störungsanfällige Bedienung gewährleistet werden.

Weiter bevorzugt ist distal vom Drehmechanismus und proximal vom Endoskopkopf ein Deflecting zum Lenken eines distalen Endoskopabschnitts bereitgestellt. Ein Deflecting ist ein zumindest zu einer Seite hin bezüglich der Schaftachse abkrümmbarer Schaftabschnitt, welcher über einen Steuerdraht betätigbar ist. Das Deflecting hat insbesondere mehrere zueinander abwinkelbar verbundene Segmente, die bevorzugt keilförmig sind. Ferner hat das Endoskop bevorzugt einen Abklappmechanismus, welcher zwischen dem Drehmechanismus, bevorzugt distal vom Deflecting, und dem Endoskopkopf angeordnet ist oder durch den Endoskopkopf gebildet wird und ein Abklappen des Endoskopkopfs bezüglich der Schaftachse ermöglicht. Der Abklappmechanismus ist bevorzugt durch einen separaten Steuerdraht, insbesondere unabhängig vom Deflecting, betätigbar. Ein maximaler Abklappwinkel des Abklappmechanismus ist bevorzugt kleiner als ein maximaler Krümmungswinkel des Deflectings.

Bevorzugt sind das erste und/ oder das zweite Seil als Antriebsseil ausschließlich zum Antrieb der Drehhülse bereitgestellt. Bevorzugt sind das erste und/ oder das zweite Seil separat von Steuerdrähten zum Antrieb einer Lenkung des Endoskopkopfs bereitgestellt, insbesondere separat zum Antrieb des Deflectings und/ oder des Abklappmechanismus. Bevorzugt sind das erste und das zweite Seil an einer im proximalen Bedienabschnitt oder Endoskopgriff bereitgestellten Seilrolle geführt und bedienbar. Vorzugsweise sind das erste und das zweite Seil an ihren proximalen Enden miteinander verbunden.

Bevorzugt sind die sämtliche Teile des Drehmechanismus derart aneinander montiert, dass diese eine unabhängig vom Schaft und/oder dem Endoskopkopf zusammenhängende Baugruppe bilden. Somit kann der Drehmechanismus vormontiert und als fertige Baugruppe einfach und kostengünstig am Schaft und am distalen Endoskopabschnitt befestigt werden.

### Figurenbeschreibung

Nachfolgend wird die vorliegende Offenbarung anhand von bevorzugten Ausführungsformen beschrieben. Diese sind jedoch nur veranschaulichender Natur und sollen den Schutzumfang der vorliegenden Offenbarung nicht einschränken. Ferner werden bei der Beschreibung der verschiedenen Ausführungsformen für gleiche Bauteile dieselben Bezugszeichen verwendet.
Fig. 1 zeigt eine perspektivische Darstellung eines offenbarungsgemäßen Endoskops.
Fig. 2 zeigt einen distalen Endoskopabschnitt des Endoskops nach Fig. 1 sowie eine Draufsicht auf einen Endoskopkopf des Endoskops.
Figen. 3, 4 und 5 zeigen schematisch einen Drehmechanismus des Endoskops nach Fig. 1 und 2 in verschiedenen Montagezuständen.
Fig. 6 zeigt eine Drehhülse des Drehmechanismus gemäß Fig. 3.
Fig. 7 zeigt eine Basishülse des Drehmechanismus nach einer zweiten Ausführungsform.
Fig. 8 zeigt schematisch eine Anordnung eines Schafts und einer Basishülse.
Fig. 9 zeigt eine perspektivische Darstellung der einzelnen Bauteile des Drehmechanismus nach einer dritten Ausführungsform.
Fig. 10 zeigt den Drehmechanismus nach Fig. 8 im zusammengebauten Zustand.

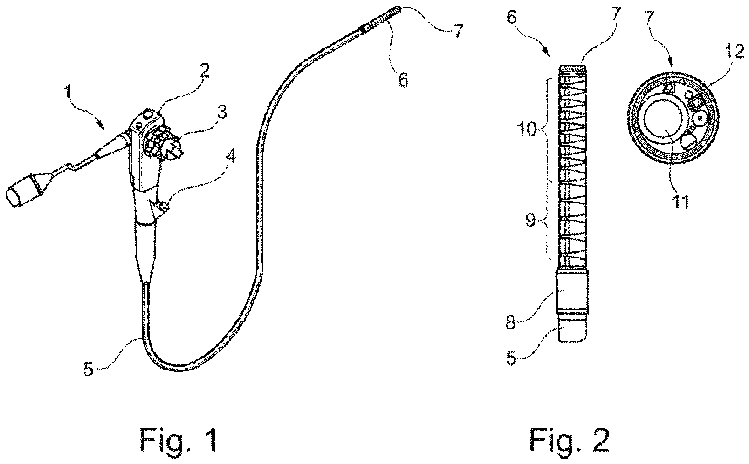

Fig. 1 zeigt eine perspektivische Darstellung eines offenbarungsgemäßen Endoskops 1. Das dargestellte Endoskop 1 hat einen proximalen Endoskopbedienabschnitt oder Endoskopgriff 2 mit Bedienelementen oder -rädern 3 und einem Luer-Anschluss 4. Vom Endoskopbedienabschnitt oder Endoskopgriff 2 erstreckt sich ein flexibler Schaft 5 in distaler Richtung. Ein distaler Endoskopabschnitt 6 ist steuerbar, um einen Endoskopkopf 7 zu verschwenken und zu drehen, welcher ein distales Ende des Endoskops 1 bildet.

Fig. 2 zeigt den distalen Endoskopabschnitt 6 des Endoskops nach Fig. 1. An einem distalen Ende des Schafts 5 ist ein offenbarungsgemäßer Drehmechanismus 8 montiert, welcher nachstehend mit Verweis auf die übrigen Figuren genauer beschrieben wird. Ein distales Ende des Drehmechanismus 8 ist mit einem Deflecting 9 verbunden, welches, wie hier beispielhaft dargestellt, mehrere keilförmige Segmente hat, die an ihrer breiten Seite aneinander angelenkt sind und zu ihrer schmalen Seite hin zusammengeklappt werden können, um das Deflecting 9 zu krümmen. Am distalen Ende des Deflectings 9 ist ein Abklappmechanismus 10 montiert, welcher, wie hier beispielhaft dargestellt, mehrere keilförmige Segmente hat, die einen größeren Keilwinkel aufweisen als die Segmente des Deflectings 9, um einen engeren Krümmungswinkel als das Deflecting 9 zu erreichen. Die Segmente des Abklappmechanismus 10 sind über die Verbindungsplatte miteinander und mit dem Deflecting 9 verbunden. Das Deflecting 9 und der Abklappmechanismus 10 können auch anders ausgebildet sein. Am distalen Ende des Abklappmechanismus 10 ist der Endoskopkopf 7 montiert. Alternativ kann der Abklappmechanismus 10 als Teil des Endoskopkopfs 7 betrachtet werden. Fig. 2 zeigt ferner eine Draufsicht auf den Endoskopkopf 7 des Endoskops 1. Dieser bildet einen Ausgang eines Arbeitskanals11 sowie weitere Elemente wie z.B. eine Kamera oder Optik 12.

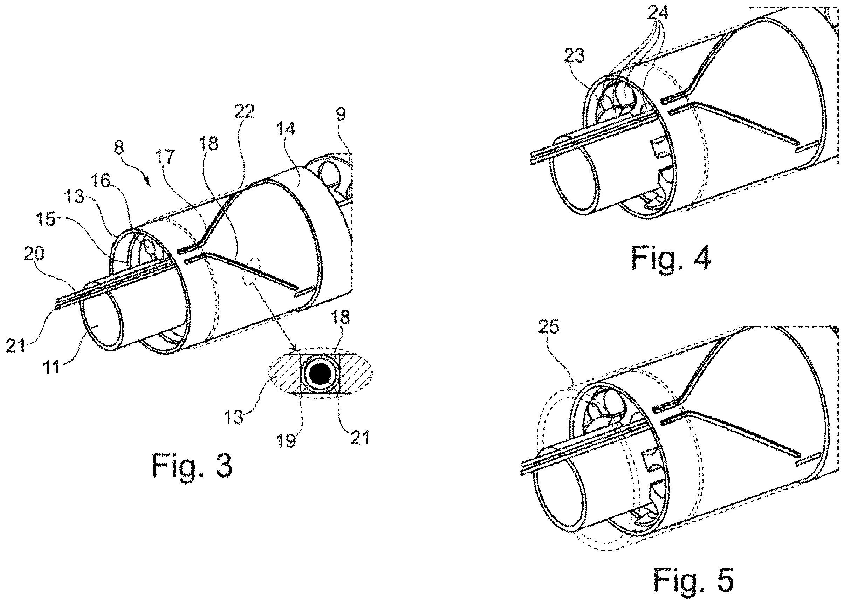

Fig.3, 4 und 5 zeigen den Drehmechanismus 8 nach Fig. 1 und 2 in verschiedenen Montagezuständen, wobei aus Übersichtlichkeitsgründen in Fig. 4 und 5 jeweils nur Bezugszeichen von zusätzlichen, in den vorherigen Figuren (d.h. Fig. 3 bzw. Fig. 3 und 4) nicht dargestellten Elementen eingefügt sind. Die übrigen Elemente entsprechen den in den vorherigen Figuren dargestellten Elementen. Der Drehmechanismus 8 hat eine Basishülse 13, welche zur Montage am distalen Ende des Schafts 5 ausgebildet ist. In die Basishülse 13 eingesteckt und drehbar dazu gelagert ist eine Drehhülse 14, deren distales Ende über ein distales Ende der Basishülse 13 hinausragt und zur Montage am Deflecting 9 ausgebildet ist.

An einem proximalen Abschnitt einer Innenseite der Basishülse 13 ist ein Halte- oder Positionierungsring 15 axialfest angeordnet, insbesondere befestigt, z.B. eingeklebt. Der Halte- oder Positionierungsring 15 ist proximal von der Drehhülse 14 oder von einer Stufe der Drehhülse 14 angeordnet ist, um einen Anschlag für die Drehhülse 14 in proximaler Richtung zu bilden. D.h., die Drehhülse 14 kann in ein distales Ende der Basishülse 13 eingesteckt werden, und zwar höchstens bis ein proximales Ende oder die Stufe der Drehhülse 14 an dem Halte- oder Positionierungsring 15 anschlägt.

Die Drehhülse bildet Axialkanäle oder Öffnungen 16, durch welche der Arbeitskanal 11 (in Fig. 3, 4 und 5 ist ein Teil davon als ein rohrförmiges Zwischenstück dargestellt) sowie ggf. weitere Leitungen 32 (in Fig. 3, 4 und 5 nicht dargestellt), sind in der Drehhülse 14 lagefest gehalten und in Axialrichtung durch die Drehhülse 14 hindurchgeführt.

Die Basishülse 13 bildet einen ersten Führungsabschnitt 17 in Form einer nut- oder schlitzförmigen Aufnahme, deren proximaler Endabschnitt 17a bevorzugt parallel zu einer Schaftlängsachse des Schafts 5 verläuft. Weiter distal macht der erste Führungsabschnitt 17 bzw. die nut- oder schlitzförmige Aufnahme einen Knick und verläuft anschließend in seinem/ ihrem mittleren Abschnitt und distalen Endabschnitt 17b schräg, d.h. er/ sie erstreckt sich distal entlang und in einer ersten Umfangsrichtung um die Basishülse 13. Ferner bildet die Basishülse 13 einen zweiten Führungsabschnitt 18 in Form einer nut- oder schlitzförmigen Aufnahme, dessen/ deren proximaler Endabschnitt 18a bevorzugt parallel zu der Schaftlängsachse des Schafts 5 verläuft. Weiter distal macht der zweite Führungsabschnitt 18 bzw. die nut- oder schlitzförmige Aufnahme einen Knick und verläuft anschließend in seinem/ ihrem mittleren Abschnitt und distalen Endabschnitt 18b schräg nach distal und in einer zweiten Umfangsrichtung entgegen der ersten Umfangsrichtung derart, dass er sich vom ersten Führungsabschnitt 17 entfernt. D.h. der erste Führungsabschnitt 17 und der zweite Führungsabschnitt 18 sind im Wesentlichen Y-förmig zueinander angeordnet, wobei die proximalen Endabschnitte 17a, 18a der ersten und zweiten Führungsabschnitte 17, 18 einen Fuß der Y-Form bilden und die mittleren Abschnitte und distalen Endabschnitte 17b, 18b der ersten und zweiten Führungsabschnitte 17, 18 Arme der Y-Form bilden.

In den ersten und zweiten Führungsabschnitten 17, 18 ist jeweils ein Kapillarrohr 19 befestigt, wie in Fig. 3 in einer Detailansicht des zweiten Führungsabschnitts 18 schematisch veranschaulicht ist. An einem distalen Ende und/ oder einem proximalen Ende des ersten und/ oder des zweiten Führungsabschnitts 17, 18 ist jeweils ein proximaler bzw. distaler Durchgang ausgebildet, welcher den jeweiligen Führungsabschnitt 17, 18 nach radial innen ablenkt oder öffnet, um die darin geführten ersten bzw. zweiten Seile 20, 21 nach radial innen zu führen, wie nachstehend genauer beschrieben wird. Die jeweiligen proximalen und/oder distalen Durchgänge können dadurch gebildet sein, dass das entsprechende distale und/ oder proximale Ende des ersten und/ oder zweiten Führungsabschnitts 17, 18 frei von den Kapillarrohren 19 ist, sodass die jeweilige nut- oder schlitzförmige Aufnahme der ersten und/ oder zweiten Führungsabschnitte 17, 18 zumindest mit der Innenumfangsfläche der Basishülse 13 verbunden ist. Alternativ können die jeweiligen proximalen und/oder distalen Durchgänge dadurch gebildet sein, dass Abschnitte der Kapillarrohre 19 an den distalen und/ oder proximalen Enden des ersten und/ oder zweiten Führungsabschnitts radial nach innen gebogen sein.

Ein erstes Seil 20 ist durch den Schaft 5 geführt und von einer Innenseite der Basishülse 13 ausgehend durch den dort vorgesehenen proximalen Durchgang in das im ersten Führungsabschnitt 17 gehaltene Kapillarrohr 19 eingeführt. Das erste Seil 20 wird durch den ersten Führungsabschnitt 17 in Umfangsrichtung umgelenkt und tritt am distalen Ende des ersten Führungsabschnitts 17 durch den dort vorgesehenen distalen Durchgang zur Innenseite der Basishülse 13 hin aus, um dort die Drehhülse 14 zu erreichen, wie später genauer beschrieben ist. Ein zweites Seil 21 ist entsprechend dem ersten Seil 20 durch den zweiten Führungsabschnitt 18 in Richtung distal und zur Drehhülse 14 geführt. Distale Endabschnitten des ersten und des zweiten Seils 20, 21 sind an der Drehhülse 14 befestigt und sind auf bzw. von diese/r auf- und abwickelbar, wie später genauer beschrieben wird.

Radial außen ist eine Deckfolie oder Deckhülse 22 bereitgestellt, welche radial außen auf der Basishülse 13 sitzt und die ersten und zweiten Führungsabschnitte von radial außen zumindest teilweise abdeckt.

In Fig. 4 ist an der Anordnung aus Fig. 3 zusätzlich eine Sperrplatte oder Abschlussscheibe 23 montiert, welche proximal zum Halte- oder Positionierungsring 15 in die Basishülse 13 eingesetzt ist und drehfest mit der Drehhülse 14 verbunden, z.B. verschraubt, ist. Der Halte- oder Positionierungsring 15 bildet einen Anschlag für die Sperrplatte oder Abschlussscheibe 23 in distaler Richtung. D.h., wenn die Drehhülse 14 mit der Sperrplatte oder Abschlussscheibe 23 verbunden ist, wird eine Axialbewegung der Drehhülse 14 relativ zur Basishülse 13 durch den Halte- oder Positionierungsring 15 im Wesentlichen verhindert. Somit ist die Drehhülse 14 zusammen mit der Sperrplatte oder Abschlussscheibe 23 in der Basishülse 13 drehbar gelagert und über den Halte- oder Positionierungsring 15 in Axialrichtung lagefestgelegt. Der Drehmechanismus 8 bildet somit eine vormontierbare Baugruppe. Die proximalen Durchgänge der ersten und zweiten Führungsabschnitte 17, 18 sind proximal von der Sperrplatte oder Abschlussscheibe 23 angeordnet oder werden teilweise durch diese definiert. Die Sperrplatte oder Abschlussscheibe 23 bildet ferner Durchführöffnungen 24 für die Leitungen 32 und den Arbeitskanal 11, um deren Lage in der Drehhülse 14 festzulegen.

In Fig. 5 ist an der Anordnung aus Fig. 4 zusätzlich ein Verbindungsring oder Übergangskörper 25 bereitgestellt, welcher proximal vom Basiskörper 13 angeordnet ist, um diesen mit dem Schaft 5 zu verbinden, z.B. über Klemmung, Verschraubung, Klebung oder dergleichen. Im vorliegenden Beispiel deckt der Verbindungsring oder Übergangskörper 25 die proximalen Durchgänge der ersten und zweiten Führungsabschnitte 17, 18 ab und liegt an seinem distalen Ende direkt an der Deckfolie oder Deckhülse 22 an.

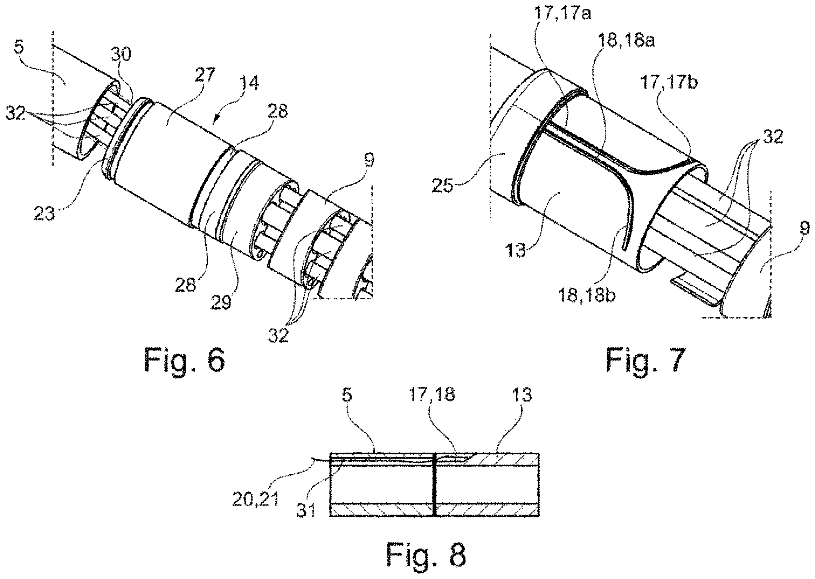

Fig. 6 zeigt die Drehhülse 14 des Drehmechanismus 8 in ihrer Lage zwischen dem Schaft 5 und dem Deflecting 9 sowie die durch diese drei Bauteile hindurchgeführten Leitungen 32. Die Drehhülse 14 kann die Drehhülse nach Fig. 3 sein, kann aber auch zu einer zweiten Ausführungsform gehören. Proximal an der Drehhülse 14 ist die Sperrplatte oder Abschlussscheibe 23 befestigt. An ihrem proximalen Ende hat die Drehhülse 14 einen Positionierabschnitt 26 mit einem Durchmesser, welcher bezüglich einem distal daran angrenzenden Lagerungsabschnitt 27 und der Sperrplatte oder Abschlussscheibe 23 verringert ist, um eine Aufnahme und Anschläge für den Halte- oder Positionierungsring 15 bereitzustellen. Über den Lagerungsabschnitt 27 ist die Drehhülse 14 an der Basishülse 13 drehbar gelagert. Distal vom Lagerungsabschnitt ist eine umlaufende, ringförmige Antriebsrille oder Ringnut 28 ausgebildet. Diese ist zur Aufnahme und Befestigung des ersten und zweiten Seils 20, 21 ausgebildet, welche somit in der Antriebsrille oder Ringnut 28 verlaufend um/ von die/der Drehhülse 14 auf- und abgewickelt werden können. Die Seile 20, 21 können z.B. in hier schematisch als Punkt dargestellte Radialbohrungen in der Antriebsrille oder Ringnut 28 eingeklebt sein.

Ein distales Ende der Drehhülse 14 bildet einen Befestigungsabschnitt 29, welcher zur Befestigung des Deflectings 9 ausgebildet ist. Zwischen der Antriebsnut oder Ringnut 28 und dem Befestigungsabschnitt 29 ist ein Kragen 30 ausgebildet, welcher an seiner distalen Seite eine Montageposition für das Deflecting 9 und an seiner proximalen Seite eine Montageposition für die Deckfolie oder Deckhülse 22 definiert. Proximal zur Drehhülse 14, d.h. innerhalb eines distalen Abschnitts des Schafts 5 und ggf. innerhalb eines proximalen Abschnitts der Basishülse 13, sind die Leitungen 32 in eine Gleitfolie 30 gehüllt, um deren Relativbewegung, d.h. eine Drehung und Bewegung in Umfangsrichtung aufgrund der Drehung der Drehhülse 14, bezüglich feststehender Bauteile des Schafts 5 bzw. im Schaft 5 zu verbessern.

Fig. 7 zeigt eine Basishülse 14 und einen Verbindungsring oder Übergangskörper 25 des Drehmechanismus 8 nach der zweiten Ausführungsform. Diese Basishülse 14 unterscheidet sich von der Basishülse 14 der ersten Ausführungsform im Wesentlichen durch die Form der ersten und zweiten nut- oder rillenförmigen Führungsabschnitte 17, 18, welche nut- oder schlitzförmige Aufnahmen sind. Diese verlaufen, wie in Fig. 7 dargestellt, in einem proximalen und einem mittleren Bereich der Führungsabschnitte 17, 18 im Wesentlichen parallel zueinander und zur Schaftachse und krümmen sich erst an ihrem distalen Endbereich in Umfangsrichtung. An ihren distalen Enden verlaufen die Führungsabschnitte 17, 18 bzw. die nut- oder schlitzförmigen Aufnahmen im Wesentlichen genau in Umfangsrichtung, sodass die Führungsabschnitte 17, 18 im Wesentlichen eine 90°-Kurve beschreiben.

Fig. 8 zeigt schematisch eine Anordnung des Schafts 5 relativ zur Basishülse 13 in einem Längsschnitt, welcher durch ein proximales Ende des ersten oder zweiten Führungsabschnitts 17, 18 verläuft. Der Schaft 5 bildet zumindest an seinem distalen Endbereich eine rillen-, nur- oder schlitzförmige Seilaufnahme 31 aus, in welcher das erste und zweite Seil 20, 21 aufgenommen sind. Somit stören die Seile 20, 21 nicht eine Bewegung der Leitungen 32 und des Arbeitskanals 11 innerhalb des Schafts 5. Die Seile können ferner innerhalb des Schafts 5 in einem gemeinsamen oder zwei separaten Hüllen geführt sein.

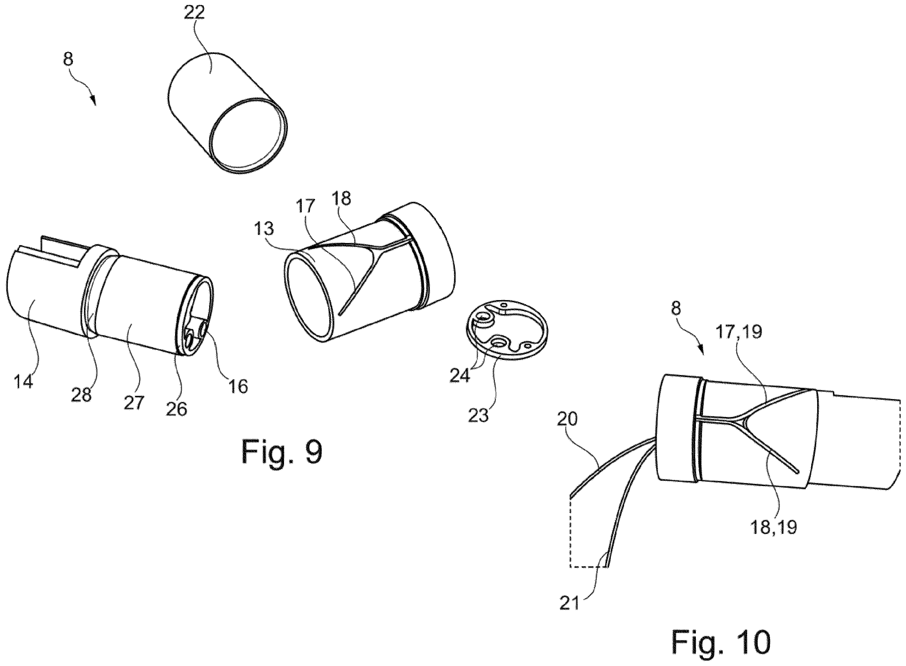

Fig. 9 zeigt eine perspektivische Darstellung der einzelnen Bauteile des Drehmechanismus nach einer dritten Ausführungsform. Ein Unterschied dieser Ausführungsform zur ersten Ausführungsform liegt darin, dass kein gesonderter Verbindungsring oder Übergangskörper 25 bereitgestellt ist, sondern dieser integral mit der Basishülse 13 ausgebildet ist. Ferner sind die ersten und zweiten Führungsabschnitte 17, 18 bzw. die nut- oder schlitzförmigen Aufnahmen an ihrem proximalen, parallel zur Schaftachse verlaufenden Enden als eine einzelne, gemeinsame nut- oder schlitzförmige Aufnahme ausgebildet, die sich erst weiter distal Y-förmig in die ersten und zweiten Führungsabschnitte 17, 18 aufteilt. Ferner sind die Führungsabschnitte 17, 18 bzw. die nut- oder schlitzförmigen Aufnahmen abgesehen von den proximalen und distalen Durchgängen radial innenseitig geschlossen.

Fig. 10 zeigt den Drehmechanismus nach Fig. 8 im zusammengebauten Zustand. Die ersten und zweiten Seile 20, 21 sind in die Führungsabschnitte 17, 18 bzw. in die darin gehaltenen oder befestigten Kapillarrohre eingeführt.

### Referenzzeichenliste

- 1: Endoskop
- 2: Endoskopbedienabschnitt oder Endoskopgriff
- 3: Bedienelementen oder -rädern
- 4: Luer-Anschluss
- 5: flexibler Schaft
- 6: distaler Endoskopabschnitt
- 7: Endoskopkopf
- 8: Drehmechanismus
- 9: Deflecting
- 10: Abklappmechanismus
- 11: Arbeitskanal
- 12: Kamera oder Optik
- 13: Basishülse
- 14: Drehhülse
- 15: Halte- oder Positionierungsring
- 16: Leitungen / Öffnungen für Leitungen
- 17: ersten Führungsabschnitt
- 17a: proximaler Endabschnitt des ersten Führungsabschnitts
- 17b: distaler Endabschnitt des ersten Führungsabschnitts
- 18: zweiten Führungsabschnitt
- 18a: proximaler Endabschnitt des ersten Führungsabschnitts
- 18b: distaler Endabschnitt des ersten Führungsabschnitts
- 19: Kapillarrohr
- 20: erstes Seil
- 21: zweites Seil
- 22: Deckfolie oder Deckhülse
- 23: Sperrplatte oder Abschlussscheibe
- 24: Durchführöffnungen
- 25: Verbindungsring oder Übergangskörper
- 26: Positionierabschnitt
- 27: Lagerungsabschnitt
- 28: Antriebsrille oder Ringnut
- 29: Befestigungsabschnitt
- 30: Gleitfolie / Gleithülle
- 31: Seilaufnahme
- 32: Leitungen

## Patentansprüche

1. Endoskop (1) mit
einem im Wesentlichen schlauchförmigen Schaft (5), welcher zum Einführen in einen Patientenkörper ausgebildet ist und sich entlang einer Schaftachse erstreckt,
einem Endoskopkopf (7), welcher distal vom Schaft (5) angeordnet ist, bezüglich der Schaftachse abkrümmbar oder abklappbar ist und eine Kamera (12) sowie einen Ausgang eines Arbeitskanals (11) aufweist,
einem Drehmechanismus (8), welcher zwischen dem Endoskopkopf (7) und dem Schaft (5) angeordnet und dazu konfiguriert ist, den Endoskopkopf (7) bezüglich des Schafts (5) um die Schaftachse zu drehen, mit
- einer Basishülse (13) welche bezüglich der Schaftachse drehfest mit dem Schaft (5) verbunden ist,
- einer Drehhülse (14), welche bezüglich der Schaftachse drehfest mit dem Endoskopkopf (7) oder einem zwischenliegenden Abschnitt verbunden ist, wobei die Drehhülse (14) und die Basishülse (13) ineinander geschachtelt sind, und
- einem ersten Seil (20) mit einem distalen Seilabschnitt, welcher an der Drehhülse (14) derart befestigt ist, dass das erste Seil (20) um die Drehhülse (14) aufwickelbar oder abwickelbar ist
**dadurch gekennzeichnet, dass** die Basishülse (13) in ihrer Seitenwand oder an einer der Drehhülse (14) radial gegenüberliegenden Fläche der Seitenwand einen ersten Führungsabschnitt (17) in Form einer rillen- oder schlitzförmigen Aufnahme bildet, in welchem das erste Seil (20) geführt ist, und an einem distalen Ende des ersten Führungsabschnitts (17) einen ersten distalen Durchgang ausbildet, durch welches das erste Seil (20) aus dem ersten Führungsabschnitt (17) zur Drehhülse (1) geführt ist.

2. Endoskop nach Anspruch 1, wobei das erste Seil (20) in einem Kapillarrohr (19) geführt ist, welches im oder am ersten Führungsabschnitt (17) der Basishülse (13) im Wesentlichen lagefest angebracht ist.

3. Endoskop (1) nach Anspruch 1 oder 2, wobei zumindest ein Abschnitt des ersten Führungsabschnitts (17) unmittelbar proximal zum ersten Durchgang schräg oder quer zur Schaftachse verläuft.

4. Endoskop (1) nach einem der Ansprüche 1 bis 3, wobei ein zweites Seil (21) bereitgestellt ist, welches durch einen zweiten Führungsabschnitt (18) in Form einer rillen- oder schlitzförmigen Aufnahme in der Basishülse (13) in Richtung distal verlaufend geführt ist und in einer zum ersten Seil (20) entgegengesetzten Umfangsrichtung um die Drehhülse (14) gewickelt oder wickelbar ist.

5. Endoskop (1) nach Anspruch 4, wobei die Basishülse (13) am distalen Ende des zweiten Führungsabschnitts (18) einen zweiten distalen Durchgang bildet, welcher in Umfangsrichtung zum ersten Durchgang beabstandet ist und durch welchen das zweite Seil (21) hindurchgeführt ist.

6. Endoskop (1) nach Anspruch 5, wobei der zweite Führungsabschnitt (18) zumindest in einem Abschnitt unmittelbar proximal zum zweiten distalen Durchgang schräg oder quer zur Schaftachse verläuft, insbesondere mit einer zum ersten Führungsabschnitt (17) entgegengesetzten Neigung oder Richtung.

7. Endoskop (1) nach einem der Ansprüche 1 bis 6, wobei die Drehhülse (14) oder die Basishülse (13) an einer bestimmten Axialposition, welche einer Axialposition der ersten und zweiten Durchgänge entspricht, eine Antriebsrille oder Ringnut (28) ausbildet.

8. Endoskop (1) nach einem der Ansprüche 1 bis 7, wobei die Basishülse (13) eine radial äußere Hülse ist und die Drehhülse (14) eine radial innere Hülse ist.

9. Endoskop (1) nach einem der Ansprüche 1 bis 8, wobei der Schaft (5) zumindest an seinem distalen Ende eine nut-, rillen- oder schlitzförmige Seilaufnahme (31) aufweist, in welchem zumindest das erste Seil (20) geführt ist.

10. Endoskop (1) nach einem der Ansprüche 1 bis 9, ferner mit elektrischen, hydraulischen und/ oder leeren Leitungen (32), welche sich durch den Schaft (5) und den Drehmechanismus (8) zum oder durch den Endoskopkopf (7) erstrecken, wobei ein distales Ende des Schafts (5) einen Hohlraum bildet, welcher derart dimensioniert ist, dass die Leitungen (32) zwischen verschiedenen, von einer Drehstellung der Drehhülse (14) abhängigen Positionen beweglich sind.

11. Endoskop (1) nach Anspruch 10, wobei der Drehmechanismus (8) angrenzend an den Schaft (5) einen Halte- oder Positionierungsring (15) bereitstellt, welcher eine Lage der Leitungen (32) bezüglich der Drehhülse (14) bestimmt.

12. Endoskop (2) nach einem der Ansprüche 10 oder 11, wobei die Leitungen (32) in einem distalen Schaftabschnitt in Umfangsrichtung beweglich sind und mit einer Gleitschicht oder einer Gleithülle (30) ummantelt sind

13. Endoskop (1) nach einem der Ansprüche 1 bis 12, wobei distal vom Drehmechanismus (8) und proximal vom Endoskopkopf (7) ein Deflecting (9) zum Lenken eines distalen Endoskopabschnitts (6) bereitgestellt ist und ferner ein Abklappmechanismus (10) zwischen dem Deflecting (9) und dem Endoskopkopf (7) angeordnet ist oder durch den Endoskopkopf (7) gebildet wird, wobei der Abklappmechanismus (10) unabhängig vom Deflecting (9) betätigbar ist, um den Endoskopkopf (7) bezüglich der Schaftachse zu einer Seite hin abzuklappen.

## Claims

1. An endoscope (1) comprising
a substantially tubular shaft (5) configured to be inserted into a patient's body and extending along a shaft axis,
an endoscope head (7) arranged distally of the shaft (5), which is bendable or foldable with respect to the shaft axis and has a camera (12) and an outlet of a working channel (11),
a rotating mechanism (8) arranged between the endoscope head (7) and the shaft (5) and configured to rotate the endoscope head (7) with respect to the shaft (5) about the shaft axis, comprising
- a base sleeve (13) which is non-rotatably connected to the shaft (5) with respect to the shaft axis,
- a rotating sleeve (14) which is non-rotatably connected to the endoscope head (7) or an intermediate portion with respect to the shaft axis, wherein the rotating sleeve (14) and the base sleeve (13) are nested into each other, and
- a first cable (20) having a distal cable portion attached to the rotating sleeve (14) such that the first cable (20) can be wound or unwound around the rotating sleeve (14),
**characterized in that** the base sleeve (13) forms, in its side wall or on a surface of the side wall radially opposite the rotating sleeve (14), a first guide portion (17) in the form of a groove-shaped or slot-shaped receptacle in which the first cable (20) is guided, and forms, at a distal end of the first guide portion (17), a first distal passage through which the first cable (20) is guided from the first guide portion (17) to the rotating sleeve (1).

2. The endoscope according to claim 1, wherein the first cable (20) is guided in a capillary tube (19) which is mounted substantially fixed in position in or on the first guide portion (17) of the base sleeve (13).

3. The endoscope (1) according to claim 1 or 2, wherein at least a portion of the first guide portion (17) directly proximal to the first passage extends obliquely or transversely to the shaft axis.

4. The endoscope (1) according to one of claims 1 to 3, wherein a second cable (21) is provided which is guided by a second guide portion (18) in the form of a groove-shaped or slot-shaped receptacle in the base sleeve (13) extending in the distal direction and is wound or can be wound around the rotating sleeve (14) in a circumferential direction opposite to the first cable (20).

5. The endoscope (1) according to claim 4, wherein the base sleeve (13) forms, at the distal end of the second guide portion (18), a second distal passage which is spaced apart from the first passage in the circumferential direction and through which the second cable (21) is guided.

6. The endoscope (1) according to claim 5, wherein the second guide portion (18) extends obliquely or transversely to the shaft axis at least in a portion directly proximal to the second distal passage, in particular with an inclination or direction opposite to the first guide portion (17).

7. The endoscope (1) according to one of claims 1 to 6, wherein the rotating sleeve (14) or the base sleeve (13) forms a drive groove or annular groove (28) at a specific axial position which corresponds to an axial position of the first and second passages.

8. The endoscope (1) according to one of claims 1 to 7, wherein the base sleeve (13) is a radially outer sleeve and the rotating sleeve (14) is a radially inner sleeve.

9. The endoscope (1) according to one of claims 1 to 8, wherein the shaft (5) has, at least at its distal end, a groove-shaped, gutter-shaped or slot-shaped cable receptacle (31) in which at least the first cable (20) is guided.

10. The endoscope (1) according to one of claims 1 to 9, further comprising electrical, hydraulic and/or empty lines (32) extending through the shaft (5) and the rotating mechanism (8) to or through the endoscope head (7), wherein a distal end of the shaft (5) forms a cavity which is dimensioned such that the lines (32) are movable between different positions depending on a rotational position of the rotating sleeve (14).

11. The endoscope (1) according to claim 10, wherein the rotating mechanism (8) provides, adjacent to the shaft (5), a retaining or positioning ring (15) which determines a position of the lines (32) with respect to the rotating sleeve (14).

12. The endoscope (2) according to one of claims 10 or 11, wherein the lines (32) are movable in the circumferential direction in a distal shaft portion and are sheathed with a sliding layer or a sliding sheath (30).

13. The endoscope (1) according to one of claims 1 to 12, wherein a deflecting portion (9) for steering a distal endoscope portion (6) is provided distally of the rotating mechanism (8) and proximally of the endoscope head (7), and furthermore a folding mechanism (10) is arranged between the deflecting portion (9) and the endoscope head (7) or is formed by the endoscope head (7), wherein the folding mechanism (10) is operable independently of the deflecting portion (9) in order to fold the endoscope head (7) to one side with respect to the shaft axis.

## Revendications

1. Endoscope (1) avec
une tige (5) sensiblement tubulaire qui est conçue pour l'introduction dans le corps d'un patient et s'étend le long d'un axe de tige,
une tête d'endoscope (7) qui est disposée distalement par rapport à la tige (5), peut être courbée ou rabattue par rapport à l'axe de tige et présente une caméra (12) ainsi qu'une sortie d'un canal de travail (11),
un mécanisme de rotation (8) qui est disposé entre la tête d'endoscope (7) et la tige (5) et est configuré pour faire tourner la tête d'endoscope (7) par rapport à la tige (5) autour de l'axe de tige, avec
- une douille de base (13) qui est connectée à la tige (5) de manière non rotative par rapport à l'axe de tige,
- une douille rotative (14) qui est connectée à la tête d'endoscope (7) ou à une section intermédiaire de manière fixe en rotation par rapport à l'axe de tige, dans lequel la douille rotative (14) et la douille de base (13) sont emboîtées l'une dans l'autre, et
- un premier câble (20) avec une section de câble distale qui est fixée à la douille rotative (14) de sorte que le premier câble (20) puisse être enroulé ou déroulé autour de la douille rotative (14)
**caractérisé en ce que** la douille de base (13) forme dans sa paroi latérale ou sur une surface de la paroi latérale opposée radialement à la douille rotative (14) une première section de guidage (17) sous forme d'un logement en forme de gorge ou de fente, dans lequel le premier câble (20) est guidé, et forme à une extrémité distale de la première section de guidage (17) un premier passage distal à travers lequel le premier câble (20) est guidé de la première section de guidage (17) à la douille rotative (1).

2. Endoscope selon la revendication 1, dans lequel le premier câble (20) est guidé dans un tube capillaire (19) qui est monté de manière sensiblement fixe en position dans ou sur la première section de guidage (17) de la douille de base (13).

3. Endoscope (1) selon la revendication 1 ou 2, dans lequel au moins une section de la première section de guidage (17) s'étend en biais ou transversalement à l'axe de tige de manière directement proximale par rapport au premier passage.

4. Endoscope (1) selon l'une quelconque des revendications 1 à 3, dans lequel est fourni un second câble (21) qui est guidé à travers une seconde section de guidage (18) sous forme d'un logement en forme de gorge ou de fente dans la douille de base (13) en s'étendant dans la direction distale et est enroulé ou peut être enroulé autour de la douille rotative (14) dans une direction périphérique opposée au premier câble (20).

5. Endoscope (1) selon la revendication 4, dans lequel la douille de base (13) forme, à l'extrémité distale de la seconde section de guidage (18), un second passage distal qui est espacé du premier passage dans la direction périphérique et à travers lequel le second câble (21) est guidé.

6. Endoscope (1) selon la revendication 5, dans lequel la seconde section de guidage (18) s'étend, au moins dans une section directement proximale par rapport au second passage distal, en biais ou transversalement par rapport à l'axe de tige, en particulier avec une inclinaison ou une direction opposée à la première section de guidage (17).

7. Endoscope (1) selon l'une quelconque des revendications 1 à 6, dans lequel la douille rotative (14) ou la douille de base (13) forme une gorge d'entraînement ou une rainure annulaire (28) au niveau d'une position axiale déterminée qui correspond à une position axiale des premier et second passages.

8. Endoscope (1) selon l'une quelconque des revendications 1 à 7, dans lequel la douille de base (13) est une douille radialement extérieure et la douille rotative (14) est une douille radialement intérieure.

9. Endoscope (1) selon l'une quelconque des revendications 1 à 8, dans lequel la tige (5) présente au moins à son extrémité distale un logement de câble (31) en forme de rainure, de gorge ou de fente, dans lequel au moins le premier câble (20) est guidé.

10. Endoscope (1) selon l'une quelconque des revendications 1 à 9, avec en outre des conduites électriques, hydrauliques et/ou vides (32) qui s'étendent à travers la tige (5) et le mécanisme de rotation (8) vers ou à travers la tête d'endoscope (7), dans lequel une extrémité distale de la tige (5) forme une cavité qui est dimensionnée de sorte que les conduites (32) soient mobiles entre différentes positions dépendant d'une position de rotation de la douille rotative (14).

11. Endoscope (1) selon la revendication 10, dans lequel le mécanisme de rotation (8) fournit une bague de maintien ou de positionnement (15) adjacente à la tige (5), laquelle bague détermine une position des conduites (32) par rapport à la douille rotative (14).

12. Endoscope (2) selon l'une quelconque des revendications 10 ou 11, dans lequel les conduites (32) sont mobiles dans une section de tige distale dans la direction périphérique et sont enveloppées d'une couche de glissement ou d'une douille de glissement (30).

13. Endoscope (1) selon l'une quelconque des revendications 1 à 12, dans lequel un déflecteur (9) est fourni de manière distale par rapport au mécanisme de rotation (8) et de manière proximale par rapport à la tête d'endoscope (7) pour diriger une section d'endoscope distale (6), et en outre un mécanisme de rabattement (10) est disposé entre le déflecteur (9) et la tête d'endoscope (7) ou est formé par la tête d'endoscope (7), dans lequel le mécanisme de rabattement (10) peut être actionné indépendamment du déflecteur (9) pour rabattre la tête d'endoscope (7) vers un côté par rapport à l'axe de tige.
